# EUROPÄISCHE PATENTSCHRIFT

(11) **EP 0 597 058 B1**
(45) Veröffentlichungstag und Bekanntmachung des Hinweises auf die Patenterteilung: **18.12.1996**
(21) Anmeldenummer: 93909749.9
(22) Anmeldetag: 28.05.1993
(51) Int. Cl.: A61M 39/00, A61F 5/44, F16K 7/04

(54) **SELBSTSCHLIESSENDES KATHETERVENTIL**
SELF-CLOSING CATHETER VALVE
VALVE DE CATHETER A FERMETURE AUTOMATIQUE

(30) Priorität: 02.06.1992 CH 1766/92; 11.09.1992 CH 2877/92
(43) Veröffentlichungstag der Anmeldung: 18.05.1994
(73) Patentinhaber: PROFIFORM AG, 6043 Adligenswil (CH); PHARMELAN HOLDING AG, 6052 Hergiswil (CH)
(72) Erfinder: SCHÖNBÄCHLER, Peter, CH-6044 Udligenswil (CH); HUSER, Peter, CH-6045 Meggen (CH); HUSER, Thomas, CH-6045 Meggen (CH); HORAT, Othmar, CH-6033 Buchrain (CH)
(74) Vertreter: Feldmann, Clarence Paul
(86) Internationale Anmeldenummer: CH9300140
(87) Internationale Veröffentlichungsnummer: WO9324173

(56) Entgegenhaltungen:
- EP-A- 0 008 064
- EP-A- 0 150 666
- EP-A- 0 455 478

## Beschreibung

Die vorliegende Erfindung betrifft ein selbstschliessendes Katheterventil gemäss Oberbegriff des Patentanspruches 1.

Katheterventile sind in sehr vielen Ausführungsformen bekannt und auf dem Markt erhältlich. Für die vorliegende Erfindung interessieren lediglich die extrakorpural angewendeten Ventile. Rein beispielsweise wird hierbei auf die folgenden Publikationen hingewiesen: EP-A-0'455'478, DE-A-39'09'63.4 oder DE-A-40'29'05.3.

Die in der Urologie angewendeten Katheterventile werden immer häufiger für chronische Patienten eingesetzt. Insbesondere in der Geriatrie müssen Patienten Katheterventile über Jahre hinweg verwenden. Diesem Umstand wurde bis heute kaum Rechnung getragen. Berücksichtigt man jedoch diese Aspekte, so kommt man zu einem Anforderungsprofil, welches mindestens die nachfolgenden Bedingungen enthält:
1. Das Ventil muss ergonometrisch geformt sein, so dass der Patient auch, wenn er lange bettlägerig ist, sich daran nicht wund reibt;
2. Da bei Langzeitpatienten die Pflegekosten besonders wesentlich sind, muss das Katheterventil äusserst preiswert sein;
3. Geriatrische Patienten sind in ihrer Motorik oftmals reduziert, was bedingt, dass das Katheterventil äusserst einfach und mit nur einer Hand bedienbar sein muss;
4. Die Langzeitverwendung setzt voraus, dass das gesamte Ventil ohne Demontage durchspülbar und das gesamte Ventil sterilisierbar ist.

Um diese Bedingungen zu erfüllen, müssen sowohl formgeberische Aspekte berücksichtigt werden, wie auch konstruktive Aspekte, die von der Formgebung teilweise wiederum diktiert werden.

Bei der vorliegenden Erfindung ging man aus von einem Katheterventil gemäss der EP-B-0'088'871. Dieses selbstschliessende Katheterventil besteht aus einem Gehäuse mit einem konischen Schlauchaufsteckstutzen, auf dem ein Katheterschlauch aufschiebbar ist und einem Ventilschlauchstück, welches das Gehäuse mindestens teilweise durchsetzt, sowie aus einem Betätigungsorgan, mittels dem das Ventil in seine Offenstellung bringbar ist. Dieses bekannte Katheterventil arbeitet mit einem Schlauchaufsteckstutzen, der im Gehäuse verschiebbar gelagert ist. Durch die Verschiebung wird das Ventilschlauchstück geknickt und abgequetscht. Die selbsttätige Schliessfunktion des Katheterventiles wird durch eine Druckfeder erreicht, welche über den Schlauchaufsteckstutzen im Bereich innerhalb des Gehäuses über den Stutzen gestülpt ist. Zur Betätigung muss der Patient diese beiden Teile auseinanderziehen gegen die Kraft der Druckfeder. Dies lässt sich mit einer Hand nicht bewerkstelligen. Die Unhandlichkeit der Zweihand-Bedienung wurde versucht dadurch zu reduzieren, dass der Schlauchaufsteckstutzen im offenen Zustand des Ventiles gegenüber dem Gehäuse verdrehbar ist und damit in der Offenstellung arretierbar. Damit ist jedoch dieses Katheterventil nicht mehr selbstschliessend. Es musste immer erst aus der Arretierungsposition bewegt werden, bevor der Selbstschliess-Effekt eintritt. Aus diesen konstruktiven Bedingungen heraus musste das Katheterventil zwangsläufig zylindrisch gestaltet sein.

Der Nachteil der Zweihand-Bedienung wurde erkannt und das Katheterventil so geändert, dass eine Einhand-Bedienung möglich wurde. Auf dem Markt ist dieses modifizierte Katheterventil erhältlich, welches einen am Gehäuse gelagerten Kipphebel aufweist, mittels dem der Schlauchaufsteckstutzen aus dem Gehäuse gegen die Kraft der Feder verschoben werden kann. An diesem Hebel reiben sich jedoch die Langszeitpatienten schnell wund.

Hinzu kommt, dass das bereits ursprünglich relativ, komplexe Ventil, welches bereits sieben Bestandteile aufwies, durch diese Modifikation noch weitere Bauteile benötigt.

Aus der EP-A-0150666 ist ein Katheterventil gemäß Oberbegriff des Patentanspruchs 1 bekannt. Hier wird der Katheterschlauch zwischen einem Steg und einem unter Federdruck gehaltenen Klemmplatte geschlossen gehalten. Mittels einer Drucktaste laßt sich die Feder Zusammendrücken und die Klemmplatte vom Steg weg bewegen um das Ventil zu öffnen. Während man die Drucktaste mit einer Hand bedient, kann man mit der anderen Hand einen die Drucktaste konzentrisch umgebenden Ring drehen und so das Ventil in die Offenstellung sichern. Die Drucktaste befinden sich an der Breitseite des Ventilgehäuses.

Es ist folglich die Aufgabe der vorliegenden Erfindung, ein Katheterventil gemäss Oberbegriff des Patentanspruches zu schaffen, welches die vorgenannten Bedingungen erfüllt und die aufgezeigten Nachteile des Ventiles gemäss Stand der Technik vermeidet.

Diese Aufgabe erfüllt ein selbstschliessendes Katheterventil mit den Merkmalen des kennzeichnenden Teiles des Patentanspruches 1.

Es ist sinnvoll, wenn das Katheterventil auch verwenbar ist zum Einsatz mit einem Urinauffangbeutel, was eine ständige Oeffnung des Ventiles voraussetzt. Die Varianten nach den Ansprüchen 3 und 4 zeigen Lösungen hierzu auf. Die Lösung nach Anspruch 3 hat insbesondere den Vorteil, dass hiermit der Schlauch während der Lagerung des Katheterventiles entlastet werden kann, das heisst, dass die Feder während dieser Zeit nicht auf den Schlauch drückt.

In der Zeichnung sind die beiden Ausführungsvarianten im Detail dargestellt und anhand der nachfolgenden Beschreibung erläutert. Es zeigt:
- Figur 1: eine Aufsicht auf das erfindungsgemässe Katheterventil in Blickrichtung auf den Schlauchaufsteckstutzen;
- Figur 2: einen teilweisen Längsschnitt durch das Ventil gemäss Figur 1 entlang der Linie A-A;
- Figur 3: einen ebensolchen Längsteilschnitt entlang der Linie B-B gemäss Figur 1, sowie
- Figur 3a: das Federlement des Katheterventiles in perspektivischer Darstellung;
- Figuren 4 und 5: Aufsicht und Querschnitt durch eine Kappe des Katheterventiles mit integriertem weiblichen Steckerteil;
- Figuren 6 und 7: Aufsicht und Seitenansicht eines arretierbaren betätigungsorganges und
- Figuren 8 und 9: Aufsicht und Frontansicht des im Betätigungsorgan nach Figuren 6 und 7 einsetzbaren Arretierungsschiebers.
- Figuren 10 und 11: zeigen eine besonders gestaltete Feder mit Halterung in der unbelasteten und in der betätigten Stellung.

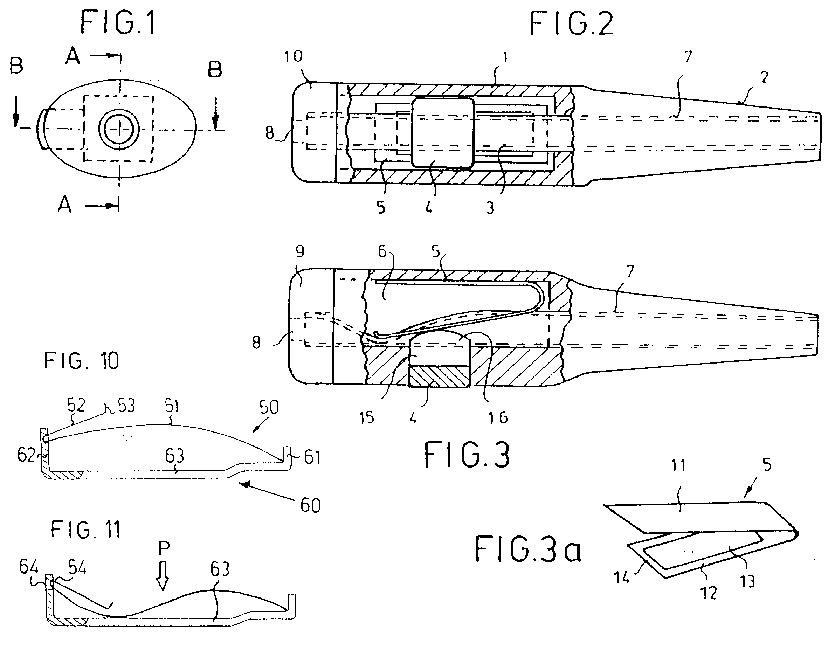

Die für das erfindungsgemässe Katheterventil bedeutungsvolle äussere Gestaltungsform geht aus den Figuren 1-3 hervor. Da diese Gestalt bei der zweiten Ausführungsform im wesentlichen übereinstimmt, wird hierauf nur einmal eingegangen. Diese äussere Gestalt des Katheterventiles besteht aus einem zylindrischen Gehäuse 1 mit einer ovalen Querschnittsform, welches bezüglich der äusseren Kontur in eine konische Spitze mit kreisförmigem Querschnitt übergeht. Der Uebergang erfolgt unter Vermeidung irgendwelcher scharfliniger Kanten.

Der konisch zulaufende Teil bildet den Schlauchaufsteckstutzen 2. Auf diesen lässt sich ein Katheterschlauch aufschieben. Ein Betätigungsorgan 4 ist an einer der beiden Schmalseiten des Gehäuses 1 angeordnet. Das Betätigungsorgan 4 ist in der Gestalt eines Druckknopfes realisiert. Ein derartig gestaltetes Katheterventil bildet keine Gefahr, dass sich der Patient hieran wund reiben kann. Die Formgebung bewirkt auch, dass das Katheterventil immer auf seiner Breitseite aufliegt. Da aber gleichzeitig das Betätigungsorgan 4 sich auf der Schmalseite befindet, ist eine unbeabsichtigte Betätigung des Ventiles, beispielsweise dadurch, dass der Patient selber auf das Ventil liegt, ausgeschlossen. Durch die Formgebung ist auch die Betätigung für den Patienten offensichtlich. Da am Katheterventil äusserlich nur das Betätigungsorgan 4 als beweglichen Teil erkennbar ist, wird der Patient das Katheterventil automatisch korrekt bedienen. Lediglich durch die farbliche Gestaltung des Betätigungsorganges 4 kann dieses auch noch optisch hervorgehoben werden.
Auch bezüglich der inneren Querschnittsform ist das Katheterventil unabhängig vom Verschliessmechanismus, entsprechend der beiden erfindungsgemässen Varianten, gestaltet. Der konische Schlauchaufsteckstutzen 2 wird von einer zylindrischen Bohrung durchsetzt und mündet in einer rechteckigen Kammer 6, die vollständig im ovalen Gehäuse 1 liegt. Die rechteckige Kammer 6 wird von einer Abschlusswand 9 begrenzt, die eine Auslassöffnung 8 aufweist, welche mit der einlassseitigen zylindrischen Bohrung 7 fluchtet.

Nachfolgend wird nun zuerst die Variante gemäss den Figuren 2 und 3 näher beschrieben. In dieser Variante sind der Schlauchaufsteckstutzen 3 und das Gehäuse 1 aus einem Stück gefertigt und die Kammer 6 wird durch eine aufsetzbare Kappe 10, welche die Abschlusswand 9 bildet, verschlossen. Auf die Gestaltung der Verbindung zwischen Kappe 10 und Gehäuse 1 wird nicht weiter eingegangen. Entsprechende Möglichkeiten sind in der kunststoffverarbeitenden Industrie in diversen Varianten bekannt. Ein Ventilschlauchstück 3, welches vorzugsweise aus einem Silikonschlauch gefertigt ist, durchsetzt das Katheterventil vom Eingang des Schlauchaufsteckstutzens 2 durch die Kammer 6 bis in die Abschlusswand 9. In der Kammer 6 liegt ein Federelement 5, welches in der Figur 3a in perspektivischer Darstellung gezeigt ist. Das Federelement 5 ist hier eine V-förmig gebogene Feder, welche einen vollflächigen Schenkel 11 und einen zweiten Schenkel 12 mit einer Ausnehmung 13 aufweist. Die Breite der Ausnehmung 13 entspricht mindestens annähernd dem äusseren Durchmesser des Ventilschlauches 3. Die Ausnehmung 13 wird an dem der Federbiegestelle gegenüberliegenden Ende von einem Steg 14 begrenzt. Der Steg 14 ist gerundet, um die Gefahr einer Verletzung des Ventilschlauches 3 zu vermeiden. Im montierten Zustand liegt der vollflächige Schenkel 11 des Federelementes 5 auf jener Wand der Kammer 6 an, welche dem Betätigungsorgan 4 gegenüberliegt. Der Ventilschlauch 3 ist unter dem unterbrochenen Schenkel 12 des Federelementes 5 hindurchgeführt. Dabei wölbt das Ventilschlauchstück sich teilweise durch die Ausnehmung 13. Der gerundete Steg 14 drückt im unbetätigten Zustand des Katheterventiles unter Vorspannung auf das Ventilschlauchstück 3.
Das Betätigungsorgan 4 ist im Prinzip als Druckknopf gestaltet, der zwei distanzierte, parallel verlaufende und sich bis in die Kammer 6 erstreckende Schenkel 15 aufweist. die beiden Schenkel 15 liegen seitlich der Durchbrechung 13 auf dem Schenkel 12 des Federelementes 5 an. Der Hubweg des Betätigungsorganes 4 ist durch in der Zeichnung nicht dargestellte Formmittel begrenzt. Das Federelement 5 hat folglich eine doppelte Funktion. Zum einen quetscht es das Ventilschlauchstück 3 zu, in dem es mit seinem Steg 14 das Ventilschlauchstück an jene Wand der Kammer 6 presst, welche vom Betätigungsorgan 4 durchdrungen wird. Gleichzeitg aber schiebt es das Betätigungsorgan 4 immer wieder in seine Ausgangsposition zurück. Damit sich das Betätigungsorgan 4 nicht in der Wandung des Gehäuses 1 verkantet, sind die Stirnflächen 16 der Schenkel 15 gewölbt. Durch Druck auf das Betätigungsorgan 4 wird folglich der Schenkel 12 mit seinem Steg 14 von der Wandung, an der das Ventilschlauchstück 3 anliegt, weggepresst. Hierdurch wird der volle Querschnitt des Ventilschlauchstückes 3 freigegeben. Lässt man das Betätigungsorgan 4 los, wird es selbsttätig in seine Ausgangsposition zurückgeschoben und das Ventilschlauchstück 3 wieder zugequetscht.

Das Federelement 5 lässt sich einfach aus einem Federstahl fertigen, wobei allerdings andere Materialien nicht ausgeschlossen sind. Muss nach einer gewissen Zeit das Ventilschlauchstück 3 ersetzt werden, so muss lediglich die Kappe 10 entfernt werden und das Schlauchstück lässt sich herausziehen. Damit das Federelement 5 nicht unbeabsichtigterweise hierbei aus der Kammer 6 herausgleitet, lassen sich in der Kammer selber entsprechende Nocken anformen oder eine Verbindung mit einem lösbaren Kleber vorsehen. Auf gleiche Weise lässt sich das Katheterventil zur vollständigen Sterilisation äusserst einfach zerlegen.

Das beschriebene Federelement 5 gemäss den Figuren 2-3a hat eine geradlinige Federcharakteristik. Mit zunehmender Verformung steigt auch die dazu erforderliche Kraft. Will der Patient das selbstschliessende Ventil zum Wasserlösen offen halten, so muss er während dieser Zeit den maximalen Druck über das Betätigungsorgan 4 auf die Feder 5 ausüben. Dies ist für betagte, oftmals geschwächte Patienten mühsam.

Die Figuren 10 und 11 zeigen ein anderes geformtes Federelement 50, welches in einer Halterung 60 liegt. Federelemente 50 und Halterung 60 lassen sich einfach in die Kammer 6 einlegen. Die formstabile Halterung 60 hat im wesentlichen die Form eines weiten U's, dessen beiden parallelen Schenkel relativ kurz sind. Zwischen den beiden schenkeln 61 und 62 ist das Federelement 50 mit Vorspannung gehalten. Die Vorspannung ergibt sich dadurch, dass das Federelement haarnadelförmig gebogen ist und somit einen längeren Betätigungsfederarm 51 und einen kürzeren Schliessfederarm 52 aufweist, wobei die Länge des Betätigungsfederarmes 51 grösser als das Bett 63 der Halterung ist. Der Betätigungsfederarm 51 überwölbt das Bett 63 und liegt einerseits in der Biegung zwischen Bett 63 und dem kürzeren Schenkel 61 und zum andern mit der haarnadelförmigen Biegung 54 in einer Ausstanzung 64.

Erfolgt mittels des hier nicht dargestellten Betätigungsorgans 4 ein Druck P auf den Betätiungsfederarm 51, so wird sich der Federarm noch geringfügig verlängern können, um dann in eine S-förmig geschwungene Form um zu schnappen. In dieser Form, gemäss Figur 11, lässt sich das Federelement mit geringer Kraft P halten. Erst wenn der Patient das Betätigungsorgan 4 vollständig loslässt, schnappt es in die Ausgangslage zurück. Der kürzere Schliessfederarm 52 schwenkt dabei verstärkt mit. Im belasteten Zustand des Federelementes gemäss Figur 11 wird somit der oberhalb des Federelementes 50 verlaufende Schlauch vollständig frei gegeben, während im unbelasteten Zustand des Federelementes 50 das nach oben abgewinklete Ende 53 des Schliessfederarmes 52 den Schlauch an die Kammerwand über dem Federelement unter Vorspannung drückt.

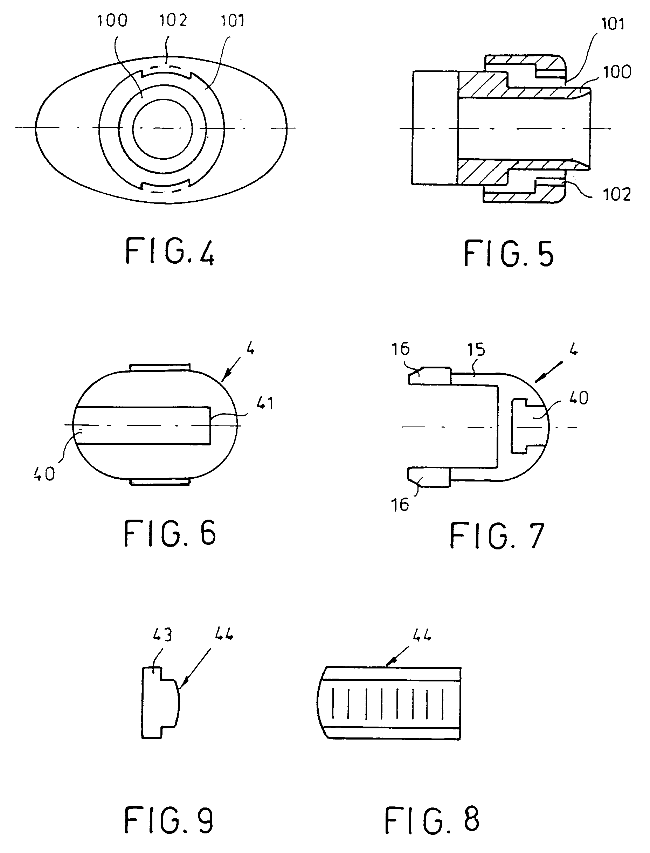

In den bisher beschriebenen Ausführungsform handelt es sich beim Katheterventil, um ein solches, das vom Patienten selber bedienbar ist, und für diesen Zweck als selbstschliessende Variante ausgestaltet ist. Für die Benutzung mit einem Urinauffangbeutel werden üblicherweise keine selbstschliessenden Katheterventile verwendet. Dies obwohl viele Patienten tatsächlich tagsüber mit einem selbsttätigbaren, nicht mit einem Urinbeutel verbundenen Katheter auskommen würden, belässt man sie mit einem nicrit selbsttätigbaren Katheterventil, damit nicht für die Nacht das Katheterventil gewechselt und ein Urinbeutel angeschlossen werden muss. Hier schafft die Ergänzung Abhilfe durch verschiedene Mittel.
Zum einen wird die Kappe 10 entsprechend den Figuren 4 und 5 speziell ausgestaltet, zum anderen wird das Betätigungsorgan 4 mit einem zusätzlichen Verriegelungsschieber versehen. Die Kappe 10, die am Urinaustrittsende des Katheterventiles 10 angeordnet ist, weist eine Durchführungstülle 100 auf, die einstückig mit der Kappe 10 aus Kunststoff gefertigt wird. Sie reicht nur geringfügig über die Kappe 10 hinaus und wird im Gebrauch von einem männlichen Kupplungsstecker, der über einen Schlauch mit dem Urinbeutel verbunden ist, überstülpt. Hierzu ist die Tülle 100 von einem ringförmigen Kanal 101 umgeben. Dieser Kanal entspricht in der Dimension dem männlichen Stecker, der in der Zeichnung nicht dargestellt ist. Zwei an der äusseren Kanalwandung angeordnete, einander diametral gegenüberliegendene Vorsprünge 102 ermöglichen eine Verriegelung des männlichen Steckers im weiblichen Stecker. Entsprechend hat der männliche Stecker radial nach aussen abstehende Vorsprünge, die nach Drehung der beiden Steckerteile relativ zueinander zu einer Verriegelung führen. Dies entspricht einer Bajonettkupplung. Die für die korrekte Verriegelung erforderliche achsiale Kraft in der gekuppelten Lage wird durch den aus dem männlichen Steckerteil leicht hervorragenden Silikonschlauch bewirkt, der in dieser Lage leicht komprimiert wird.
Die so gestaltete Kappe kann tagsüber ohne Schlauchanschluss und Stecker direkt als Urinaustritt dienen, wobei die Tülle 100 einen sauberen Austritt garantiert. Für die Nacht braucht lediglich ein Urinbeutel mit Schlauch, an dem der männlichen Stecker befestigt ist, angekuppelt werden.
In der Nacht sollte nun das Katheterventil ständig offen sein. Dies lässt sich einfach realisieren, wenn das Betätigungsorgan 4, gemäss den Figuren 6 und 7 ausgestaltet und mit einem Verriegelungsschieber, nach Figuren 8 und 9 ergänzt ist.
In der Aufsicht nach Figur 6 erkennt man deutlich, dass im Betätigungsorgan 4, in der Form einer Druckknopfes, eine Bahn 40 eingelassen ist, die einseitig von einem Anschlag 41 begrenzt ist. In der Figur 7 erkennt man deutlich, dass diese Bahn 4' im Querschnitt die Form einer T-Nut hat. Bei der Montage des Druckknopfes 4 muss darauf geachtet werden, dass der Anschlag 41 auf der Seite zum Deckel 10 hin liegt. Der Verriegungsschieber 44, der in der T-nutförmigen Bahn 40 liegt, lässt sich somit nur in Richtung von der Kappe 10 weg verschieben. Die Kappe 10 liegt an der vom Patienten weg weisenden Seite. Drückt er auf das Betätigungsorgan 4, so hat er üblicherweise immer die Neigung dabei, eine von sich weg gerichtete Kraftkomponente auszuüben. Diese bewirkt, dass der Verriegelungsschieber zum Anschlag 41 gedrückt wird, so dass keine ungewollte Verriegelung in die Oeffnungslage resultiert. Das Katheterventil verbleibt somit in der selbsttätig schliessenden Position. Wird das Ventil jedoch vom Pflegepersonal betätigt, was immer dann der Fall ist, wenn der Urinbeutel für die Nacht angehängt oder ausgewechselt werden muss, so führt zwar diese Person auch eine Druckbewegung mit einer Bewegungskomponente von sich weg aus, doch ist diese Bewegungskomponente zum Patienten gerichtet und somit automatisch zur gewünschten Verriegelungsposition führend.

Ein bedeutender Vorteil dieses Verriegelungs- und Urinbeutel-Ankopplungssystems ist, dass der Urinbeutel angehängt und ausgewechselt werden kann, ohne dass das Ventil hierzu geöffnet werden muss. Damit ist die Gefahr einer eventuellen Kontamination drastisch verringert.

## Patentansprüche

1. Selbstschliessendes Katheterventil, bestehend aus einem Gehäuse (1) mit einem konischen Schlauchaufsteckstutzen (2) auf den ein Katheterschlauch aufschiebbar ist, und einem Ventilschlauchstück (3), welches das Gehäuse (1) mindestens teilweise durchsetzt, sowie aus einem Betätigungsorgan (4) mittels dem das Ventil in seine Offenstellung bringbar ist, wobei das Ventilgehäuse (1) einen ovalen Querschnitt aufweist, und dass das Betätigungsorgan (4), welches auf ein Federelement (5) wirkt, mindestens annähernd vollständig innerhalb der Aussenkontur des Gehäuses befindet und durch eine rein translatorische Bewegung in Richtung der Längsachse des Querschnittovals bedienbar ist, und dass im Gehäuse eine Kammer (6) sich befindet, in dem das Federelement (5,50) durch Verformung des Ventilschlauchstückes dieses schliesst, dadurch gekennzeichnet, dass das Federelement (5) aus einer V-förmig gebogenen Feder besteht, dessen einen Schenkel (11) vollflächig ist und an der Wandung der Kammer (6) in seiner ganzen Länge aufliegt, während der andere, bewegliche Schenkel (12) vorzugsweise eine Ausnehmung (13) in der Breite des Ventilschlauchstückes (3) aufweist, welche Ausnehmung von einem endständigen Steg (14) begrenzt ist, mit dem der Ventilschlauch (3) an der gegenüberliegenden Wand der Kammer (6) quetschbar ist, wobei das an der Schmalseite des Gehäuses angeordnete Betätigungorgan (4) ein Knopf ist, welcher auf zwei, den endständigen Steg (14) tragenden Längsstege neben der Ausnehmung (13) auf den beweglichen Schenkel (12) wirkt.

2. Selbstschliessendes Katheterventil nach Anspruch 1, dadurch gekennzeichnet, dass das Gehäuse (1) aus zwei Kunststoffteile besteht, wobei der eine Teil den Schlauchaufsteckstutzen (2) und die Kammer (6) umfasst, während der andere, damit verbundene Teil eine Kappe (10) ist, welche den Hohlraum (6) abschliesst und eine Durchführung (8) als Harnaustrittsöffnung aufweist, in dem das Ventilschlauchstück (3) endseitig gehalten ist.

3. Selbstschliessendes Katheterventil nach Anspruch 1, dadurch gekennzeichnet, dass die dem Schlauchaufsteckstutzen (2) gegenüberliegende Gehäusewand einen Steckerteil (100-102) zur Verbindung mit einem passenden Schlauchstecker aufweist.

4. Selbstschliessendes Katheterventil nach Anspruch 3, dadurch gekennzeichnet, dass der Steckerteil im Gehäuse eine zentrische Tülle (100) mit umgebenden Ringkanal (101), in den radial nach innen gerichtete Vorsprünge zur bajonettartigen Verriegelung vorgesehen sind, aufweist.

5. Selbstschliessendes Katheterventil nach Anspruch 1, dadurch gekennzeichnet, dass das Betätigungsorgan (4) mit einem zusätzlichen im Betätigungsorgan (4) verschieblich (40) gelagerten Verriegelungsschieber (44) versehen ist, mit dem das Betätigungsorgan in der eingeschränkten, die Offenstellung des Ventiles bewirkenden Lage arretierbar ist.

6. Selbstschliessendes Katheterventil nach Anspruch 5, dadurch gekennzeichnete dass im Betätigungsorgan eine Bahn (40), in der Form einer T-Nut eingelassen ist, welche in Richtung zum Schlauchaufsteckstutzen (2) offen und auf der gegenüberliegenden Seite einen Anschlag (41) aufweist, und in die ein Verriegelungsschieber gleitend lagert.

7. Selbstschliessendes Katheterventil nach Anspruch 1, dadurch gekennzeichnet, dass das Federelement (50) in einer Halterung (60) unter Vorspannung gehalten ist, und haarnadelförmig gebogen ist, wobei ein längerer Betätigungsfederarm (51) zwischen zwei Schenkeln (61,62) der Halterung (60) im unbelasteten Zustand vorgespannt ein Bett (63) der Halterung überwölbt und unter Druck in eine S-förmige Form umschnappt.

## Claims

1. A self-closing catheter valve, comprising a housing (1) with a conical pipe attachment stub (2) on which a catheter tube can be engaged, and a valve pipe section (3) which at least partly traverses the housing (1), together with an operating member (4) by means of which the valve can be brought into its open position, the valve housing (1) having an oval cross-section and the operating member (4), which acts on a spring component (5), is located at least approximately wholly within the outer contour of the housing and is operable by a purely translatory movement in the direction of the longitudinal axis of its oval cross-section, and within the housing is located a chamber (6), in which the spring component (5, 50) closes the valve pipe section by deformation of the latter,
characterised in that the spring component (5) comprises a spring bent in V-shaped manner and whose one leg (11) is of complete surface form and engages with its entire length against the wall of the chamber (6), whereas the other, movable leg (12) preferably has a recess (13) of the width of the valve pipe section (3), said recess being bounded by a terminal web (14) by which the valve pipe (3) can be squeezed against the opposite wall of the chamber (6), the operating member (4) located on the narrow side of the housing being a button which acts on the movable leg (12) on two longitudinal webs beside the recess (13) and carrying the terminal web (14).

2. A self-closing catheter valve according to claim 1, characterised in that the housing (1) comprises two plastics parts, one part embracing the pipe attachment stub (2) and the chamber (6), whereas the other part connected thereto is a cap (10), which terminates the cavity (6) and has a passage (8) as a urine discharge opening, in which is terminally held the valve pipe section (3).

3. A self-closing catheter valve according to claim 1, characterised in that the housing wall facing the pipe attachment stub (2) has a plug part (100-102) for connection to a matching pipe plug.

4. A self-closing catheter valve according to claim 3, characterised in that the plug part has in the housing a central nipple (100) with a surrounding annular channel (101), in which are provided radially inwardly directed projections for bayonet-type locking.

5. A self-closing catheter valve according to claim 1, characterised in that the operating member (4) is provided with an additional locking slide (44) displaceably (40) mounted in the operating member (4) and with which the latter can be locked in the restricted position bringing about the open position of the valve.

6. A self-closing catheter valve according to claim 5, characterised in that in the operating member is provided a path (40) in the form of a T-slot, which is open in the direction towards the pipe attachment stub (2) and has on the opposite side a stop (41) and in which is slidingly mounted a locking slide.

7. A self-closing catheter valve according to claim 1, characterised in that the spring component (50) is held under pretension in a fastening (60) and bent in hairpin-like manner, a longer operating spring arm (51) between two legs (61, 62) of the fastening (6) in the unloaded state arching in pretensioned form over a bed (63) of the fastening and under pressure snapping into an S-shape.

## Revendications

1. Valve de cathéter à fermeture automatique, constituée par un boîtier (1) comprenant une tubulure conique d'enfoncement du tuyau (2) sur laquelle on peut enfoncer un tuyau de cathéter, et par un tronçon de tuyau de valve (3) qui traverse le boîtier (1), du moins partiellement, ainsi que par un organe d'actionnement (4) au moyen duquel la valve peut être amenée dans sa position ouverte, cependant que le boîtier de valve (1) présente une section transversale ovale, que l'organe d'actionnement (4) qui agit sur un élément élastique (5) se trouve complètement à l'intérieur du contour extérieur du boîtier, du moins approximativement, qu'il peut être manoeuvré par un déplacement de translation pure dans la direction de l'axe longitudinal de l'ovale de la section transversale, et que se trouve dans le boîtier une chambre (6) dans laquelle l'élément élastique (5, 50) ferme le tronçon de tuyau de valve par déformation de celui-ci, caractérisée par le fait que l'élément élastique (5) est constitué par un ressort plié en forme de V dont une aile (11) est une surface pleine, celle-ci portant sur toute sa longueur sur la paroi de la chambre (6), tandis que l'autre aile mobile (12) présente de préférence un évidement (13) dont la largeur est celle du tronçon de tuyau de valve (3), cet évidement étant délimité par une barrette d'extrémité (14) au moyen de laquelle le tuyau de valve (3) peut être écrasé contre la paroi opposée de la chambre (6), cependant que l'organe d'actionnement (4) disposé sur le petit côté du boîtier est un bouton qui agit sur l'aile mobile (12) à côté de l'évidement (13), et ce, sur deux barrettes longitudinales qui portent la barrette d'extrémité (14).

2. Valve de cathéter à fermeture automatique selon la revendication 1, caractérisée par le fait que le boîtier (1) est constitué par deux parties en matière plastique, l'une de ces parties entourant la tubulure d'enfoncement du tuyau (2) et la chambre (6), tandis que l'autre partie reliée à la première est un capuchon (10) qui ferme la cavité (6), qui présente un passage (8) servant d'orifice de sortie de l'urine et dans lequel le tronçon de tuyau de valve (3) est maintenu d'un côté.

3. Valve de cathéter à fermeture automatique selon la revendication 1, caractérisée par le fait que la paroi du boîtier qui est opposée à la tubulure d'enfoncement du tuyau (2) présente une partie à enficher (100 à 102) en vue de sa liaison avec une partie de tuyau à enficher qui lui est adaptée.

4. Valve de cathéter à fermeture automatique selon la revendication 3, caractérisée par le fait que la partie à enficher présente dans le boîtier une douille centrale (100) pourvue d'un canal annulaire (101) qui l'entoure et dans lequel des parties en saillie dirigées vers l'intérieur dans le sens radial sont prévues pour un verrouillage analogue à une fermeture à baïonnette.

5. Valve de cathéter à fermeture automatique selon la revendication 1, caractérisée par le fait que l'organe d'actionnement (4) est pourvu d'un coulisseau de verrouillage supplémentaire (44) qui est monté en coulissement (40) dans l'organe d'actionnement (4) et au moyen duquel l'organe d'actionnement peut être bloqué dans la position délimitée produisant la position d'ouverture de la valve.

6. Valve de cathéter à fermeture automatique selon la revendication 5, caractérisée par le fait qu'est ménagé dans l'organe d'actionnement un guidage (40) qui se présente sous la forme d'une rainure en T, qui est ouvert dans la direction de la tubulure d'enfoncement du tuyau (2), qui présente une butée (41) sur le côté opposé et dans lequel un coulisseau de verrouillage est monté en glissement.

7. Valve de cathéter à fermeture automatique selon la revendication 1, caractérisée par le fait que l'élément élastique (50) est maintenu dans un support (60) sous une compression préalable, et qu'il est recourbé en forme d'épingle à cheveux, cependant qu'un bras élastique d'actionnement plus long (51) forme un arc au-dessus d'un fond (63) du support entre deux rebords (61, 62) du support (60) en étant comprimé au préalable à l'état non chargé, et que, sous l'effet d'une pression, il se détend en sens inverse pour prendre la forme d'un S.
